(19) Europäisches Patentamt
European Patent Office
Office européen des brevets

(11) EP 1 971 376 B1

(12) **EUROPEAN PATENT SPECIFICATION**

(45) Date of publication and mention of the grant of the patent:
**20.07.2016 Bulletin 2016/29**

(21) Application number: **06839389.1**

(22) Date of filing: **15.12.2006**

(51) Int Cl.:
***A61L 27/52*** (2006.01) ***C08F 283/12*** (2006.01)
***G02B 1/04*** (2006.01)

(86) International application number:
**PCT/US2006/047876**

(87) International publication number:
**WO 2007/078871 (12.07.2007 Gazette 2007/28)**

(54) **SILICONE CONTAINING POLYMERS FORMED FROM NON-REACTIVE SILICONE CONTAINING PREPOLYMERS**

SILIKONHALTIGE POLYMERE AUS NICHT-REAKTIVEN SILIKONHALTIGEN PRÄPOLYMEREN

POLYMÈRES CONTENANT DE LA SILICONE FORMÉS À PARTIR DE PRÉPOLYMÈRES NON RÉACTIFS CONTENANT DE LA SILICONE

(84) Designated Contracting States:
**DE FR GB IE IT**

(30) Priority: **30.12.2005 US 755371 P**
**08.12.2006 US 608642**

(43) Date of publication of application:
**24.09.2008 Bulletin 2008/39**

(73) Proprietor: **Johnson and Johnson Vision Care, Inc.**
**Jacksonville, FL 32256 (US)**

(72) Inventors:
- **ALLI, Azaam**
  **Jacksonville, FL 32277 (US)**
- **MAHADEVAN, Shivkumar**
  **Orange Park, FL 32003 (US)**
- **FORD, James D.**
  **Orange Park, Florida 32003 (US)**
- **MOLOCK, Jr., Frank F.**
  **Orange Park, Florida 32003 (US)**
- **TURNER, David C.**
  **Jacksonville, FL 32223 (US)**

(74) Representative: **Kirsch, Susan Edith et al**
**Carpmaels & Ransford LLP**
**One Southampton Row**
**London WC1B 5HA (GB)**

(56) References cited:
**EP-A1- 1 386 924** **WO-A-03/022322**
**WO-A-2004/081105** **US-A- 4 260 725**
**US-A- 4 711 943** **US-B1- 6 218 503**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention related to silicone containing polymers formed from non-reactive silicone containing prepolymers.

### Background of the Invention

The present invention relates to polymeric materials. More particularly, it relates to prepolymers which can be used to form biomedical devices without additional wetting agents.

The suitability of a material for use in biomedical devices depends on a number of factors that often include the wettability of the material and its proclivity for adhesion or reaction with biological materials such as proteins and lipids. In ophthalmic applications such as contact lenses and intraocular implants, oxygen permeability is also an important consideration.

Silicone hydrogels can be a particularly desirable material for making biomedical devices such as contact lenses because of their generally good oxygen permeability. However, their hydrophobic nature makes the devices made from them difficult to wet. One approach for dealing with this problem is to coat the hydrogels with a more hydrophilic coating. This adds an additional level of complexity to their manufacture. Additionally, coating material selection can be difficult as can the determination of proper coating thickness, coating uniformity and other factors that can affect physiological performance.

The surface properties of polymeric objects such as contact lenses may be modified by the inclusion of macromers having a hydrophobic portion, a hydrophilic portion, a chain transfer agent, and an unsaturated end group in the monomer mix used to make the objects. The macromers can include poly-N-vinylpyrrolidone having molecular weights of 500-10,000 with 1,000-5,000 being most preferred. The macromers are polymerized into the hydrogel and do improve wettability of the polymers. However, the improvement is generally not to such a degree that lenses can be made from the hydrogels without the need for a hydrophilic coating.

WO 03/022322 relates to silicone hydrogels utilised in the manufacture of contact lenses that are formed from reactive silicone containing macromers, high molecular weight hydrophilic polymers and compatibilising components.

### Summary of the Invention

The present invention includes a polymer composition as defined in the claims formed from a reactive mixture comprising, consisting and consisting essentially of (a) at least one substantially non-reactive prepolymer comprising silicone containing groups and compatibilizing groups and (b) a hydrophilic component comprising at least one amide containing monomer, provided that said reactive mixture is substantially free of reactive prepolymer.

### Description of the Invention

The polymers of the present invention are formed, as defined in the claims, from (a) at least one substantially non-reactive prepolymer comprising silicone containing groups and compatibilizing groups and (b) a hydrophilic component comprising at least one monomer capable of hydrogen bonding with said prepolymer, provided that said reactive mixture is substantially free of reactive prepolymer.

As used herein, a "biomedical device" is any article that is designed to be used while either in or on mammalian tissues or fluid, and preferably in or on human tissue or fluids. Examples of these devices include but are not limited to catheters, implants, stents, and ophthalmic devices such as intraocular lenses and contact lenses. In one embodiment the biomedical devices are ophthalmic devices, particularly ophthalmic lenses, most particularly contact lenses.

As used herein, the terms "lens" and "ophthalmic device" refer to devices that reside in or on the eye. These devices can provide optical correction, wound care, drug delivery, diagnostic functionality, cosmetic enhancement or effect or a combination of these properties. The term lens includes but is not limited to soft contact lenses, hard contact lenses, intraocular lenses, overlay lenses, ocular inserts, and optical inserts.

All percentages in this specification are weight percentages unless otherwise noted.

As used herein, the phrase. "without a surface treatment" or "not surface treated" means that the exterior surfaces of the devices of that embodiment are not separately treated to improve the wettability of the device. Examples of treatments which may be foregone because of the present invention include plasma treatments, grafting, coating and the like. However, coatings which provide properties other than improved wettability, such as, but not limited to antimicrobial coatings and the application of color or other cosmetic enhancement, may be applied to devices of the present invention.

As used herein substantially non-reactive means that the prepolymer does not covalently bond to itself or other components in the reactive mixture during curing. Thus, in one embodiment the prepolymer is substantially free from groups which are capable of forming covalent bonds under the curing and processing conditions selected to make the polymer and any desired article therefrom. In one embodiment the prepolymers of the present invention comprise less than 1% groups which are capable of forming covalent bonds under the selected curing and processing conditions.

Any reactive silicone containing component may be used to form the prepolymers of the present invention. Suitable silicone containing components contain at least one [-Si-O-Si] group, in a monomer, macromer or prepolymer. Preferably, the Si and attached O are present in the silicone-containing component in an amount greater than 20 weight percent, and more preferably greater than 30 weight percent of the total molecular weight of the silicone-containing component. Useful silicone-containing components preferably comprise at least one reactive group. Any group which is capable of reaction by any mode of reaction may be used. Examples include groups which are capable of undergoing thermal, photo or visible light initiated reaction, free radical polymerization, group transfer polymerization, condensation, esterification, atom transfer radical polymerization, ring opening polymerization, anionic polymerization, cationic polymerization, and the like. Examples of reactive groups capable of undergoing free radical polymerization include acrylate, methacrylate, acrylamide, methacrylamide, N-vinyl lactam, N-vinylamide, and styryl functional groups. Examples of silicone-containing components which are useful in this invention may be found in U.S. Pat. Nos. 3,808,178; 4,120,570; 4,136,250; 4,153,641; 4,740,533; 5,034,461 and 5,070,215, 5,998,498 and EP080539. Other types of reactive groups are known in the art and may also be used.

Examples of silicone-containing monomers which may be used as the silicone containing component are polysiloxanylalkyl(meth)acrylic monomers represented by the following formula:

Formula II

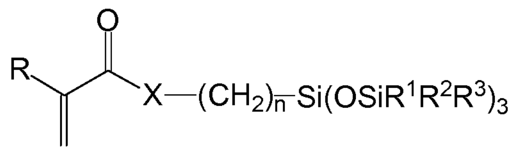

wherein: R denotes H or lower alkyl; X denotes O or $NR^4$; each $R^4$ independently denotes hydrogen or methyl, each $R^1$-$R^3$ independently denotes a lower alkyl radical or a phenyl radical, and n is 1 or 3 to 10.

Examples of these polysiloxanylalkyl (meth)acrylic monomers include methacryloxypropyl tris(trimethylsiloxy) silane, pentamethyldisiloxanyl methylmethacrylate, and methyldi(trimethylsiloxy)methacryloxymethyl silane. In some embodiments methacryloxypropyl tris(trimethylsiloxy)silane is preferred.

Another class of silicone-containing components is a poly(organosiloxane) prepolymer represented by Formula III:

Formula III

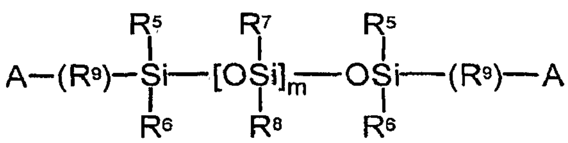

wherein each A independently denotes an activated unsaturated group, such as an ester or amide of an acrylic or a methacrylic acid or an alkyl group having 1 to 8 carbon atoms, and in some embodiments 1 to 3 carbon atoms or aryl group having 6 to 10 carbon atoms (providing that one A comprises an activated unsaturated group capable of undergoing radical polymerization); each of $R^5$, $R^6$, $R^7$ and $R^8$ are independently selected from the group consisting of a monovalent hydrocarbon radical or a halogen substituted monovalent hydrocarbon radical having 1 to 18 carbon atoms, and in some embodiment 1 to 5 carbon atoms which may have ether linkages between carbon atoms, or a monovalent siloxanyl;

$R^9$ denotes a divalent hydrocarbon radical having from 1 to 22 carbon atoms, which may be substituted ether, hydroxyl, ester functional groups, and m is 0 or an integer greater than or equal to 1, and preferable 5 to 400, and more preferably 10 to 300. One specific example is $\alpha$, $\omega$-bismethacryloxypropyl poly-dimethylsiloxane. Another example is mPDMS (monomethacryloxypropyl terminated mono-n-butyl terminated polydimethylsiloxane).

Another useful class of silicone containing components includes silicone-containing vinyl carbonate or vinyl carbamate monomers of the following formula:

Formula IV

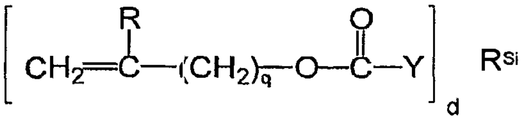

wherein: Y denotes O, S. or NH, $R^{Si}$ denotes a silicone-containing organic radical; R denotes

- $(CH_2)_qSi[(CH_2)_sCH_3]_3$ ; $(CH_2)_qSi[OSi(CH_2)_sCH_3]_3$ ;

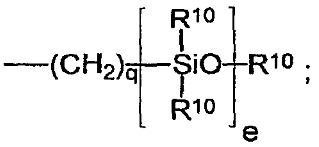

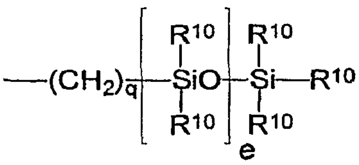

hydrogen or methyl; d is 1, 2, 3 or 4; in some embodiments d is one, and q is 0 or 1. Suitable silicone-containing organic radicals $R^{Si}$ include the following:

wherein: $R^{10}$ is an alkyl radical or a fluoroalkyl radical having 1 to 6 carbon atoms; e is 1 to 200; q is 1, 2, 3 or 4; and s is 0, 1, 2, 3,4 or 5.

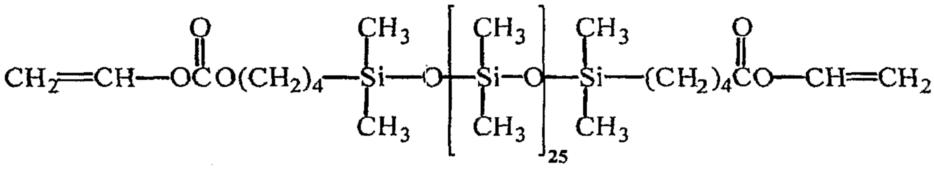

The silicone-containing vinyl carbonate or vinyl carbamate monomers specifically include: 3-[tris(trimethylsiloxy)silyl] propyl allyl carbamate; 3-[tris(trimethylsiloxy)silyl] propyl vinyl carbamate; trimethylsilylethyl vinyl carbonate; trimethylsilylmethyl vinyl carbonate.

The silicone groups in the silicone containing component may either be pendant to the main chain of the prepolymer, or may be included in the backbone of the prepolymer.

The amount of silicone containing component in the prepolymer may vary from about 10 to about 95, from about 20 to about 90 and in some embodiments from about 30 to about 80 weight %, based upon all the reactive components used to make the prepolymer.

The prepolymer also comprises at least one compatibilizing group. Suitable compatibilizing groups render the prepolymer miscible in or with the hydrophilic component. In some embodiments the compatibilizing group comprises at least one hydrogen bond participant, and in some embodiments the compatibilizing group comprises at least one hydrogen bond donating group. Compatibilizing groups are carboxyl groups, thiols, phenols, amides of primary amines, ammonia, ureas of primary amines, urethanes of primary amines, hydroxyl groups and combinations thereof. In one embodiment the compatibilizing group comprises at least one hydroxyl group, such as but not limited to mono or bis hydroxyl alkyls and polyols.

The compatibilizing group may be a part of the silicone component or it may be a separate polymerizable component. Examples of silicone components which comprise compatibilizing groups include 2-propenoic acid, 2-methyl-,2-hydroxy-3-[3-[1,3,3,3-tetramethyl-1-[(trimethylsilyl)oxy]disiloxanyl]propoxy]propyl ester, hydroxypropyl methacrylate terminated polydimethyl siloxane, (3-methacryloxy-2-hydroxypropyloxy)propyltris(trimethylsiloxy)silane, mono-(3-methacryloxy-2-hydroxypropyloxy)propyl terminated, mono-butyl terminated polydimethylsiloxanes, combinations thereof and the like.

In another embodiment the compatibilizing group is incorporated into the prepolymer via a separate compatibilizing component which comprises at least one compatibilizing group, as defined above and at least one reactive group. The reactive group may be any group which is reactive under the polymerization conditions selected for making the prepolymer. For example if the prepolymer is made via free radical polymerization, any reactive groups capable of undergoing free radical polymerization may be used. Reactive groups for other types of reaction are known to those of skill in the art.

Examples of suitable compatibilizing groups include N,N-dimethylacrylamide (DMA), 2-hydroxyethyl acrylate, 2-hydroxyethyl methacrylate, glycerol methacrylate, 2-hydroxyethyl methacrylamide, methacrylic acid, acrylic acid and hydrophilic vinyl-containing monomers such as N-vinyl lactams, including N-vinyl pyrrolidone (NVP), N-vinyl-N-methyl acetamide, N-vinyl-N-ethyl acetamide, N-vinyl-N-ethyl formamide, N-vinyl formamide, N-2-hydroxyethyl vinyl carbamate, combinations thereof and the like. In one embodiment where a compatibilizing component is used, the compatibilizing component is selected from N,N-dimethylacrylamide (DMA), 2-hydroxyethyl methacrylate, N-vinyl pyrrolidone (NVP), N-vinyl-N-methyl acetamide and combinations thereof.

The compatibilizing groups in the compatibilizing component may either be pendant to the main chain of the prepolymer, or may be included in the backbone of the prepolymer. The compatibilizing component can be dispersed randomly over the entire prepolymer structure, may be grouped on the ends of the prepolymer, or be in blocks. In some embodiments where the prepolymer is formed from compatibilizing components and silicone containing components it is preferred that the prepolymer be a random copolymer.

The amount of compatibilizing group should be sufficient to allow the prepolymer and hydrophilic component to be miscible in the selected solvent and to remain compatible during the polymerization. Suitable amounts of compatibilizing component include from about 1 to about 50 weight %, and in some embodiments from about 3 to about 25 weight % based upon the weight of all the components in the prepolymer.

The prepolymer may also comprise other components which do not detract from the desired features of the resulting prepolymer.

The silicone component and the compatiblizing component are reacted together to form the prepolymer. The prepolymer may be formed by any known polymerization reaction including free radical polymerization, anionic and cationic polymerization, group transfer polymerization, condensation, atom transfer radical polymerization, ring opening polymerization, and the like. Reaction conditions will depend upon the components selected and the desired molecular weight. For example, in one embodiment where the prepolymer components comprise free radical reactive groups, the prepolymers of the present invention may be formed by mixing the silicone component, the compatibilizing component, and a polymerization catalyst with an optional solvent, and polymerizing the mixture under conditions suitable for the selected catalyst. The reaction is run to completion so that the resulting prepolymer is substantially non-reactive. Suitable reaction times include from about 10 seconds to about 1 hour and the reaction is generally run in an inert atmosphere. Alternatively, additional components such as chain transfer agents or capping agents may be included. Prepolymers of the present invention will have weight average molecular weights between about 50,000 and about 1,000,000, in some embodiments 500,000, in some embodiments 200,000 Daltons, as measured via gel permeation chromatography using refractive index detection.

For free radical reactions, suitable polymerization catalysts are well known in the art and include thermal and photoinitiators. The polymerization initiators includes compounds such as lauryl peroxide, benzoyl peroxide, isopropyl percarbonate, azobisisobutyronitrile, and the like, that generate free radicals at moderately elevated temperatures, and photoinitiator systems such as aromatic alpha-hydroxy ketones, alkoxyoxybenzoins, acetophenones, acylphosphine oxides, bisacylphosphine oxides, and a tertiary amine plus a diketone, mixtures thereof and the like. Illustrative examples of photoinitiators are 1-hydroxycyclohexyl phenyl ketone, 2-hydroxy-2-methyl-1-phenyl-propan-1-one, bis(2,6-dimethoxybenzoyl)-2,4-4-trimethylpentyl phosphine oxide (DMBAPO), bis(2,4,6-trimethylbenzoyl)-phenyl phosphineoxide (Irgacure 819), 2,4,6-trimethylbenzyldiphenyl phosphine oxide and 2,4,6-trimethylbenzoyl diphenylphosphine oxide, benzoin methyl ester and a combination of camphorquinone and ethyl 4-(N,N-dimethylamino)benzoate. Commercially available visible light initiator systems include Irgacure 819, Irgacure 1700, Irgacure 1800, Irgacure 819, Irgacure 1850 (all from Ciba Specialty Chemicals) and Lucirin TPO initiator (available from BASF). Commercially available UV photoinitiators include Darocur 1173 and Darocur 2959 (Ciba Specialty Chemicals). These and other photoinitators which may be used are disclosed in Volume III, Photoinitiators for Free Radical Cationic & Anionic Photopolymerization, 2nd Edition by J.V. Crivello& K. Dietliker; edited by G. Bradley; John Wiley and Sons; New York; 1998. The initiator is used in effective amounts to initiate photopolymerization of the silicone containing component and compatibilizing component, e.g., from about 0.1 to about 2 parts by weight per 100 parts of the mixture. Polymerization can be initiated using the appropriate choice of heat or visible or ultraviolet light or other means depending on the polymerization initiator used. Alternatively, initiation can be conducted without a photoinitiator using, for example, e-beam. However, when a photoinitiator is used, the preferred initiators are bisacylphosphine oxides, such as bis(2,4,6-trimethylbenzoyl)-phenyl phosphine oxide (Irgacure 819®) or a combination of 1-hydroxycyclohexyl phenyl ketone and bis(2,6-dimethoxybenzoyl)-2,4-4-trimethylpentyl

phosphine oxide (DMBAPO), and the preferred method of polymerization initiation is visible light. The most preferred is bis(2,4,6-trimethylbenzoyl)-phenyl phosphine oxide (Irgacure 819®).

The prepolymer can be purified before it is combined with the hydrophilic monomer, for example by precipitating it in a solvent. Alternatively it may be possible to form the prepolymer, preferably in one of the diluents as described above, and combine this prepolymer/diluent mixture with the hydrophilic monomer without purification of the prepolymer. The formation of the prepolymer/diluent solution may be formed for example in a continuous process, for example by heating or irradiating the prepolymer precursors and diluent with the appropriate initiator. This latter method is particularly well suited to high-speed automated methods of manufacture.

The polymers are formed by reacting at least one hydrophilic component in the presence of the selected prepolymer. Suitable hydrophilic components are polymerizable and capable of hydrogen bonding with said prepolymer. In some embodiments the hydrophilic component has a Hansen Solubility Parameter $\delta$ of at least about 12, in some embodiments at least about 14 and in some embodiments, at least about 15. In some embodiments, the hydrophilic component is a hydrophilic monomer. As used herein the term "monomer" is a compound containing at least one polymerizable group and an average molecular weight of about less than 2000 Daltons, as measured via gel permeation chromatography using refractive index detection. Thus, monomers include dimers and in some cases oligomers, including oligomers made from more than one monomeric unit.

Examples of suitable hydrophilic components include hydrophilic monomers that have at least one polymerizable group and at least one hydrophilic functional group. A polymerizable group is are group capable of undergoing reaction via free radical polymerization, anionic and cationic polymerization, group transfer polymerization, condensation, atom transfer radical polymerization, ring opening polymerization, and the like. Examples of polymerizable groups with polymerizable double bonds include acrylic, methacrylic, acrylamido, methacrylamido, fumaric, maleic, styryl, isopropenylphenyl, O-vinylcarbonate, O-vinylcarbamate, allylic, O-vinylacetyl and N-vinyllactam and N-vinylamido double bonds. Such hydrophilic monomers may themselves be used as crosslinking agents if they have two or more polymerizable double bonds per molecule.

"Acrylic-type" or "acrylic-containing" monomers are those monomers containing the acrylic group ($CR^{12}H{=}CR^{13}COX$)

wherein $R^{13}$ is H or $CH_3$, $R^{12}$ is H, alkyl or carbonyl, and X is O or N, which are also known to polymerize readily, such as N,N-dimethylacrylamide (DMA), 2-hydroxyethyl acrylate, glycerol methacrylate, 2-hydroxyethyl methacrylamide, polyethyleneglycol monomethacrylate, methacrylic acid, acrylic acid and mixtures thereof.

The hydrophilic component may also be selected from hydrophilic vinyl-containing monomers such as N-vinyl lactams, including. N-vinyl pyrrolidone (NVP), N-vinyl-N-methyl acetamide, N-vinyl-N-ethyl acetamide, N-vinyl-N-ethyl formamide, N-vinyl formamide, N-2-hydroxyethyl vinyl carbamate, N-carboxy-ß-alanine N-vinyl ester, combinations thereof and the like.

Other hydrophilic monomers that can be employed in the invention include polyoxyethylene polyols having one or more of the terminal hydroxyl groups replaced with a functional group containing a polymerizable double bond. Examples include polyethylene glycol with one or more of the terminal hydroxyl groups replaced with a functional group containing a polymerizable double bond. Examples include polyethylene glycol reacted with one or more molar equivalents of an end-capping group such as isocyanatoethyl methacrylate, ("IEM"), methacrylic anhydride, methacryloyl chloride, vinylbenzoyl chloride, or the like, to produce a polyethylene polyol having one or more terminal polymerizable olefinic groups bonded to the polyethylene polyol through linking moieties such as carbamate or ester groups.

Still further examples are the hydrophilic vinyl carbonate or vinyl carbamate monomers disclosed in U.S. Pat. No.5,070,215, the hydrophilic oxazolone monomers disclosed in U.S. Pat. No. 4,910,277 and hydrophilic oxazoline monomers such as 2-ethyl-2-oxazoline. Other suitable hydrophilic monomers will be apparent to one skilled in the art.

In one embodiment the hydrophilic component comprises at least one amide or urethane containing hydrophilic monomer and in another embodiment, an amide containing monomer. Examples of suitable amide containing hydrophilic monomers include N,N-dimethyl acrylamide, (DMA), 2-hydroxyethyl methacrylamide, N-vinylpyrrolidone (NVP), N-vinyl-N-methylacetamide (VMA), N-vinylacetamide, N-vinyl-N-methylpropionamide, N-vinyl-N-methyl-2-methylpropionamide, N-vinyl-2-methylpropionamide, N-vinyl-N,N'-dimethylurea, combinations thereof and the like.

In one embodiment the hydrophile monomer comprises DMA, NVP, VMA or mixtures thereof.

The hydrophilic component should be included in an amount which provide at least about 20% and preferably at least about 25% water content to the resulting polymer when combined with the remaining components. In some embodiments, the hydrophilic component is present in amounts up to about 60 weight %, between about 10 to about 60 weight%, between about 20 to about 60 weight %, all based upon the weight of all components in the reactive mixture.

The polymers of the present invention are formed from reactive mixtures. As used herein a reactive mixture, is a mixture comprising the at least one substantially non- reactive prepolymer, a hydrophilic component and any other components necessary or desirable to provide either the reactive mixture or the finished polymer with the desired properties. Suitable additional components include, but are not limited to, UV absorbers, medicinal agents, nutraceutical agents, antimicrobial compounds, reactive tints, pigments, copolymerizable and nonpolymerizable dyes, release agents,

wetting agents, compatibilizing components, cross-linking agents, chain transfer agents, combinations thereof and the like. In another embodiment additional components include, but are not limited to, UV absorbers, medicinal agents, nutraceutical agents, antimicrobial compounds, reactive tints, pigments, copolymerizable and nonpolymerizable dyes, release agents, cross-linking agents, chain transfer agents, combinations thereof and the like.

In one embodiment the reactive mixture is substantially free from silicone components other than the prepolymer. In another embodiment, the reactive mixture comprises less than 5 weight percent, based upon the weight of all reactive components and less than 1 weight % of silicone components other than the prepolymer.

The reactive mixture may also comprise a diluent. Suitable diluents will solubilize all components in the reactive mixture. Generally an appropriate diluent may be selected by determining the Hansen Solubility Parameters of the prepolymer, hydrophilic component and diluent.

Hansen Solubility Parameters describe polymer-liquid interactions and each solvent and polymer can be assigned a set of three parameters $\delta_H$, $\delta_P$, $\delta_D$, describing their interactions. A description of the system is found in Handbook of Polymer Liquid Interaction Parameters and Solubility Parameter, CRC Press, Inc. 1990 and Handbook of Solubility Parameters and Other Cohesion Parameters, A. F. M. Barton, CRC Press, 1985, Table 5. Each set of three parameters defines a point in a three-dimensional solubility space.

For a liquid to act as a solvent for a given prepolymer/hydrophilic component combination, the HSP parameters of the solvent are desirably between to those of the prepolymer and hydrophilic component. The Hansen solubility parameters for a particular prepolymer can be determined by solubility tests in which a sample of the prepolymer is stored in a number of different solvents. By observing whether the prepolymer is dissolved, swelled or unchanged, it is possible to plot a solubility sphere for the particular prepolymer in the solubility space substantially as described in Hansen Solubility Parameters; A User's Handbook, Charles M. Hansen, pg 43-53, CRC Press 2000 and CMH's Sphere computer program for the calculations. In one embodiment the distance between the solvent and each of the prepolymer and the hydrophilic component in the three-dimensional solubility space should not exceed the following values: $\delta_D$ from about 5 to about 10, $\delta_P$ from about 4 to about 12, $\delta_H$ from about 10 to about 6. Thus, it will be appreciated that and the polarity of the prepolymers and hydrophilic components is increased, the polarity of the solvent to be used should also increase. In some embodiments it may be desirable to select the prepolymer and the hydrophilic component such that their polarities are similar.

In one embodiment, suitable diluents include those, which possess both a hydrophilic and a hydrophobic nature. The hydrophilic nature may be characterized by hydrogen donating ability, using Kamlet alpha values (also referred to as alpha values). The hydrophobic nature of the diluent may be characterized by the Hansen solubility parameter $\delta p$. Suitable diluents for the present invention are good hydrogen bond donors and have a polarity between the prepolymer and hydrophilic component. As used herein a "good" hydrogen bond donor, will donate hydrogen at least as readily as 3-methyl-3-pentanol. For certain diluents it is possible to measure the hydrogen bond donating ability by measuring the Kamlet alpha value (or as used herein "alpha value"). Suitable alpha values include those between about 0.05 and about 1 and preferably between about 0.1 and about 0.9.

It will be appreciated that the properties of the selected prepolymer and hydrophobic components may effect the properties of the diluents which will provide the desired compatibilization. For example, if the reaction mixture contains only moderately polar components, diluents having moderate $\delta p$ may be used. If however, the reaction mixture contains strongly polar components, the diluent may need to have a high $\delta p$.

Examples of specific diluents which may be used include, without limitation, 1-ethoxy-2-propanol, diisopropylaminoethanol, isopropanol, 3,7-dimethyl-3-octanol, 1-decanol, 1-dodecanol, 1-octanol, 1-pentanol, 2-pentanol, 1-hexanol, 2-hexanol, 2-octanol, 3-methyl-3-pentanol, *tert*-amyl alcohol, *tert*-butanol, 2-butanol, 1-butanol, 2-methyl-2-pentanol, 2-propanol, 1-propanol, ethanol, 2-ethyl-1-butanol, 1-*tert*-butoxy-2-propanol, 3,3-dimethyl-2-butanol, *tert*-butoxyethanol, 2-octyl-1-dodecanol, decanoic acid, octanoic acid, dodecanoic acid, 2-(diisopropylamino)ethanol mixtures thereof and the like.

Classes of suitable diluents include, without limitation, alcohols having 2 to 20 carbons, amides having 10 to 20 carbon atoms derived from primary amines and carboxylic acids having 8 to 20 carbon atoms. In some embodiments, primary and tertiary alcohols are preferred. Preferred classes include alcohols having 5 to 20 carbons and carboxylic acids having 10 to 20 carbon atoms.

Where the hydrophilic component comprises vinyl groups, suitable diluents include 3,7-dimethyl-3-octanol, 1-dodecanol, 1-decanol, 1-octanol, 1-pentanol, 1-hexanol, 2-hexanol, 2-octanol, 1-dodecanol, 3-methyl-3-pentanol, 1-pentanol, 2-pentanol, t-amyl alcohol, *tert*-butanol, 2-butanol, 1-butanol, 2-methyl-2-pentanol, 2-ethyl-1-butanol, ethanol, 3,3-dimethyl-2-butanol, 2-octyl-1-dodecanol, decanoic acid, octanoic acid, dodecanoic acid, mixtures thereof and the like.

Mixtures of diluents may be used. In some embodiments it may be advantageous to use diluents with different properties. Moreover, it should be appreciated that when mixtures are used, the mixtures may include a diluent with properties within those specified herein and diluent(s) which do not possess the defined properties, or may contain diluents which each contain only one of the specified properties.

The diluents may be used in amounts up to about 50% by weight of the total of all components in the reactive

mixture. More preferably the diluent is used in amounts less than about 45% and more preferably in amounts between about 15 and about 40% by weight of the total of all components in the reactive mixture.

When the hydrophilic components selected are free radical polymerizable, a polymerization catalyst may also be used. Any of the polymerization catalysts described above with respect to the synthesis of the prepolymer may be used.

One or more cross-linking agents, also referred to as cross-linking monomers, may also be included in the reactive mixture. Examples of suitable cross-linking agents include monomers with two or more polymerizable double bonds, such as ethylene glycol dimethacrylate ("EGDMA"), trimethylolpropane trimethacrylate ("TMPTMA"), glycerol trimethacrylate, polyethylene glycol dimethacrylate (wherein the polyethylene glycol preferably has a molecular weight up to, e.g., about 5000), and other polyacrylate and polymethacrylate esters, such as the end-capped polyoxyethylene polyols described above containing two or more terminal methacrylate moieties. The cross-linking agents are used in the usual amounts, e.g., from about 0.0004 to about 0.02 mole per 100 grams of reactive components in the reactive mixture. Alternatively, if the hydrophilic component acts as the cross-linking agent, the addition of a crosslinking agent to the reaction mixture is optional. Examples of hydrophilic monomers which can act as a crosslinking agent include polyoxyethylene polyols described above containing two or more terminal methacrylate moieties.

The polymers of the present invention are useful for the manufacture of medical devices including but not limited to ophthalmic devices, such as ophthalmic lenses and punctual plugs and particularly in some embodiments, contact lenses. The polymers of the present invention may also be used as carriers for pharmaceutical and nutraceutical compounds.

The reactive mixtures of the present invention can be formed by any of the methods know to those skilled in the art, such as shaking or stirring, and used to form polymeric articles or devices by known methods. When the prepolymer is particularly viscous, it may be necessary to blend or stir the mixture for an extended period of time to form a solution, for example from about 2 to about 100 hours. In some embodiments it may be necessary to blend or stir the mixture at elevated temperatures such as about 30 to about 70°C.

For example, the biomedical devices of the invention may be prepared by including a polymerization initiator in the reactive mixture and curing by appropriate conditions to form a product that can be subsequently formed into the appropriate shape by lathing, cutting and the like. Alternatively, the reactive mixture may be placed in a mold and subsequently cured into the appropriate article. In yet another embodiment the reactive mixture can be cast and cured into other articles such as films, fibers, sheets, plates, molded parts and articles and coatings for any of the foregoing. In yet another embodiment, the polymers of the present invention may be cured and ground for use as a carrier for active agents such as pharmaceutical and nutraceutical compounds.

Suitable polymerization conditions may be selected based upon the reactive groups on the hydrophilic component. Where the hydrophilic component comprises free radical reactive groups, an initiator is generally included. Suitable initiators include thermal and photoinitiators and are disclosed in US 6,822,016. Polymerization conditions for hydrophilic components with other reactive functionalities will be understood by those of skill in the art, with reference to the disclosure and examples of the present invention.

In one embodiment the reactive mixtures is used for the production of contact lenses. Various processes are known for processing the reactive mixture in the production of contact lenses, including spincasting and static casting. Spincasting methods are disclosed in U.S. Pat. Nos. 3,408,429 and 3,660,545, and static casting methods are disclosed in U.S. Pat. Nos. 4,113,224 and 4,197,266. The preferred method for producing contact lenses comprising the polymer of this invention is by the molding of the silicone hydrogels, which is economical, and enables precise control over the final shape of the hydrated lens. For this method, the reactive mixture is placed in a mold having the shape of the final desired silicone hydrogel, i.e., water-swollen polymer, and the reaction mixture is subjected to conditions whereby the monomers polymerize, to thereby produce a polymer/diluent mixture in the shape of the final desired product. Then, this polymer/diluent mixture is treated with a solvent to remove the diluent and ultimately replace it with water, producing a silicone hydrogel having a final size and shape which are quite similar to the size and shape of the original molded polymer/diluent article. This method can be used to form contact lenses and is further described in U.S. Pat. Nos. 4,495,313; 4,680,336; 4,889,664; and 5,039,459.

The biomedical devices, and particularly ophthalmic lenses of the present invention have a balance of properties which makes them particularly useful. Such properties include clarity, water content, oxygen permeability and contact angle. Thus, in one embodiment, the biomedical devices are contact lenses having a water content of greater than about 17%, in some embodiments greater than about 20% and in other embodiments greater than about 25%.

As used herein clarity means substantially free from visible haze. Clear lenses have a haze value of less than about 200%, and in some embodiments less than about 150%. It will be appreciated that not all articles formed for polymers of the present invention require clarity. However, for articles where clarity is important, such as ophthalmic devices, articles having the clarity recited herein may be produced.

Suitable oxygen permeabilities for silicone containing lenses are greater than about 40 barrer and in some embodiments greater than about 60 barrer.

Also, the biomedical devices, and particularly ophthalmic devices and contact lenses have dynamic contact

angles (advancing) which are less than about 90°, and in some embodiments less than about 80°. In some embodiments the articles of the present invention have combinations of the above described oxygen permeability, water content and contact angle. All combinations of the above ranges are deemed to be within the present invention.

The non-limiting examples below further describe this invention.

## Examples

Wettability is measured by measuring the dynamic contact angle or DCA, typically at 23 °C, with borate buffered saline, using a Wilhelmy balance. The wetting force between the lens surface and borate buffered saline is measured using a Wilhelmy microbalance while the sample strip cut from the center portion of the lens is being immersed into or pulled out of the saline at a rate of 100 microns/sec . The following equation is used

$$F = 2\gamma p \cos\theta \qquad \text{or} \qquad \theta = \cos^{-1}(F/2\gamma p)$$

where F is the wetting force, y is the surface tension of the probe liquid, p is the perimeter of the sample at the meniscus and $\theta$ is the contact angle. Typically, two contact angles are obtained from a dynamic wetting experiment - advancing contact angle and receding contact angle. Advancing contact angle is obtained from the portion of the wetting experiment where the sample is being immersed into the probe liquid, and these are the values reported herein. At least four lenses of each composition are measured and the average is reported.

Modulus was measured by using the crosshead of a constant rate of movement type tensile testing machine equipped with a load cell that is lowered to the initial gauge height. A suitable testing machine includes an Instron model 1122. A dog-bone shaped sample having a 0.522 inch length, 0.276 inch "ear" width and 0.213 inch "neck" width was loaded into the grips and elongated at a constant rate of strain of 2 in/min. until it broke. The initial gauge length of the sample (Lo) and sample length at break (Lf) were measured. Twelve specimens of each composition were measured and the average is reported. Tensile modulus was measured at the initial linear portion of the stress/strain curve.

Haze is measured by placing a hydrated test lens in borate buffered saline in a clear 20 x 40 x 10 mm glass cell at ambient temperature above a flat black background, illuminating from below with a fiber optic lamp (Titan Tool Supply Co. fiber optic light with 0.5" diameter light guide set at a power setting of 4-5.4) at an angle 66° normal to the lens cell, and capturing an image of the lens from above, normal to the lens cell with a video camera (DVC 1300C:19130 RGB camera with Navitar TV Zoom 7000 zoom lens) placed 14 mm above the lens platform. The background scatter is subtracted from the scatter of the lens by subtracting an image of a blank cell using EPIX XCAP V 1.0 software. The subtracted scattered light image is quantitatively analyzed, by integrating over the central 10 mm of the lens, and then comparing to a - 1.00 diopter CSI Thin Lens®, which is arbitrarily set at a haze value of 100, with no lens set as a haze value of 0. Five lenses are analyzed and the results are averaged to generate a haze value as a percentage of the standard CSI lens.

The water content was measured as follows: lenses to be tested were allowed to sit in packing solution for 24 hours. Each of three test lens were removed from packing solution using a sponge tipped swab and placed on blotting wipes which have been dampened with packing solution. Both sides of the lens were contacted with the wipe. Using tweezers, the test lens were placed in a weighing pan and weighed. The two more sets of samples were prepared and weighed as above. The pan was weighed three times and the average is the wet weight.

The dry weight was measured by placing the sample pans in a vacuum oven which has been preheated to 60°C for 30 minutes. Vacuum was applied until at least 0.4 inches Hg is attained. The vacuum valve and pump were turned off and the lenses were dried for four hours. The purge valve was opened and the oven was allowed reach atmospheric pressure. The pans were removed and weighed. The water content was calculated as follows:

$$\text{Wet weight} = \text{combined wet weight of pan and lenses} - \text{weight of weighing pan}$$

$$\text{Dry weight} = \text{combined dry weight of pan and lens} - \text{weight of weighing pan}$$

$$\%\ \text{water content} = \frac{(\text{wet weight} - \text{dry weight})}{\text{wet weight}} \times 100$$

The average and standard deviation of the water content are calculated for the samples are reported.

Oxygen permeability (Dk) was determined by the polarographic method generally described in ISO 9913-1: 1996(E), except that the measurement is conducted at an environment containing 2.1% oxygen, This environment is created by equipping the test chamber with nitrogen input at 1800 ml/min and an air input at 200 ml/min. The t/Dk is calculated using the adjusted $p_{O2}$. The resulting Dk value is reported in barrers.

The following abbreviations are used in the examples below:

| | |
|---|---|
| SiGMA | 2-propenoic acid, 2-methyl-,2-hydroxy-3-[3-[1,3,3,3-tetramethyl-1-[(trimethylsilyl)oxy]disiloxanyl]propoxy]propyl ester |
| DMA | N,N-dimethylacrylamide |
| HEMA | 2-hydroxyethyl methacrylate, |
| MPDMS | 800-1000 MW ($M_n$) monomethacryloxypropyl terminated mono-n-butyl terminated polydimethylsiloxane |
| NVP | N-vinylpyrrolidone |
| IPA | isopropyl alcohol |
| MeOH | methanol |
| EtOAc | ethyl acetate |
| D3O | 3,7-dimethyl-3-octanol |
| TEGDMA | tetraethyleneglycol dimethacrylate |
| TRIS | 3-methacryloxypropyltris(trimethylsiloxy)silane |
| CGI 819 | bis(2,4,6-trimethylbenzoyl)-phenyl phosphineoxide |
| DAROCUR 1173 | α-hydroxy-α,α-dimethylacetophenone |
| DPMA | di(propylene glycol) methyl ether acetate |

Example 1 - Polymer A

Into each of two amber-colored jars were placed 54 g mPDMS, 6 g HEMA, 200 mg CGI 819 and 50 ml hexanol. After mixing for one hour these solutions were degassed by placing them in a vacuum chamber for one hour at about 80 mm Hg, breaking the vacuum by filling the chamber with nitrogen every 15 minutes. The solutions were then irradiated from above with visible light from Philips TL03 fluorescent bulbs for one hour, in a nitrogen atmosphere at room temperature about 14 inches above the solution. The solutions were then combined, and the product was precipitated by addition of about 500 ml MeOH. The resulting upper layer was decanted and the lower layer was washed three times by addition of MeOH, shaking, and decanting the upper layer. The bottom layer was dissolved in 200 ml ethyl acetate, and precipitated with about 500 ml methanol. The upper layer was decanted, and the lower layer was washed two more times with 200 ml MeOH, then stripped of volatiles in a rotary evaporator under reduced pressure at 55°C to yield 80 g of Prepolymer A as a colorless solid.

Prepolymer A (21.1 g) was blended in 23.9 g D3O to form a Prepolymer A Solution.

Example 2

A clear solution was formed from 55.5% (wt) Prepolymer A (in solid form), 39% DMA, 4% TEGDMA, 1.5 % CGI 819 with D3O in a ratio of 70/30 (weight ratio of Prepolymer and reactive components to D3O diluent). This blend was degassed under 40 mm Hg for 20 minutes, then cured in TOPAZ front and polypropylene back contact lens molds under a Philips TL03 light for 1 hour at 50°C. The molds were opened and the lenses were released into a 50:50 (wt) solution of IPA and water. The lenses were extracted with IPA for 2 hours, then transferred (20 minutes in each) to 75:25, 50:50, 25:75 and 0:100 solutions of IPA and water. The properties of the resulting lenses are shown in Table 1, below.

Example 3

Lenses were made following the procedure of Example 2, but with the formulation and results shown in Table 1.

**Table 1**

| Component | Example 2 | Example 3 |
|---|---|---|
| Prepolymer A | 55.5 wt% | 57 wt% |
| DMA | 39 wt% | 40 wt% |
| TEGDMA | 4 wt% | 1.5 wt% |

(continued)

| Component | Example 2 | Example 3 |
|---|---|---|
| CGI 819 | 1.5 wt% | 1.5 wt% |
| Monomer diluent ratio (D3O diluent) | 70/30 | 60/40 |
| | | |
| Dynamic contact angle | 81° | * |
| Haze | 59% | * |
| Modulus | * | 40 psi |
| Dk | * | 91 |
| Water content | * | 60.1% |
| * not measured | | |

Example 4 - Prepolymer B solution

A blend of 54 g mPDMS, 5 g HEMA, 50 mg CGI 819 and 50 g t-amyl alcohol was made and placed in a nitrogen environment for several hours. It was irradiated for about 1 hour with visible light from Philips TL03 bulbs in nitrogen at room temperature about 5 inches above the solution. The resulting viscous solution was poured into 200 ml MeOH. After stirring, the upper layer was decanted and 100 ml MeOH was added to the lower layer. This mixture was again stirred and the upper layer was decanted. 240 ml IPA was added to the lower layer to form a solution. 250 ml MeOH was added and the upper layer was decanted again. 200 ml IPA was added to the lower layer to make a clear solution, followed by 400 ml MeOH to precipitate polymer, and the upper layer was again decanted. Volatiles were stripped from the lower layer on a rotary evaporator to give 21.1 g product as a clear, very viscous oil. This oil was dissolved in 23.9 g D3O to yield 45 g Prepolymer B solution.

Example 5 - Prepolymer C solution

A blend of 54 g TRIS, 5 g, HEMA, 50 mg CGI 819 and 50 g t-amyl alcohol was made and degassed for about 5 minutes by applying vacuum (30 mm Hg) and filling the flask with nitrogen, then storing the solution overnight under a nitrogen atmosphere. The solution was irradiated with Philips TL 20W/03T placed about 14 inches above the solution for 1 hour in nitrogen at room temperature. The resulting viscous solution was poured into 200 ml MeOH. After stirring, the upper layer was decanted and 100 ml MeOH was added to the lower layer. This mixture was again stirred and the upper layer was decanted. 150 ml IPA was added to form a solution. 200 ml MeOH was added and the upper layer was decanted again. 150 ml IPA was added to the lower layer followed by 300 ml MeOH, and the upper layer was again decanted. Volatiles were stripped from the lower layer on a rotary evaporator to give 29.5 g product as a clear, viscous oil. This oil was dissolved in D3O to yield a solution of 43% (wt) prepolymer and 57% D3O.

Example 6

3.35 g Prepolymer A Solution was combined with 1.10 g DMA, 40 μl TEGDMA, and 14 μl DAROCUR 1173 to form a clear blend. Lenses were formed by curing in TOPAZ/polypropylene molds for 30 minutes under Philips TL 20W/09N fluorescent UV bulbs in nitrogen at room temperature about 5 inches above the solution. The molds were opened and the lenses were released in 70/30 IPA/water, with two exchanges for fresh solution, then placed into borate buffered saline solution. The lenses were slightly opaque.

Example 7

3.35 g Prepolymer A Solution was combined with 1.10 g NVP, 40 μl TEGDMA, and 14 μl DAROCUR 1173 to form a clear blend. Lenses were formed by curing in TOPAZ/polypropylene molds for 30 minutes under Philips TL 20W/09N fluorescent UV bulbs in nitrogen at room temperature about 5 inches above the solution. The molds were opened and the lenses were released in 70/30 IPA/water, with two exchanges for fresh solution, then placed into borate buffered saline solution. The lenses were opaque.

Example 8

3.35 g Prepolymer B solution was combined with 1.10 g DMA, 40 μl TEGDMA, and 14 μl DAROCUR 1173 to form a clear blend. Lenses were formed by curing in TOPAZ/polypropylene molds for 30 minutes under Philips TL 20W/09N fluorescent UV bulbs. The molds were opened and the lenses were released in 70/30 IPA/water, with two exchanges for fresh solution, then placed into borate buffered saline solution. The lenses were opaque.

Example 9 - SiGMA Prepolymer Solution

When a degassed solution of 29.5 g SiGMA, 25 mg CGI 819 and 25 g t-amyl alcohol was irradiated with visible light, an insoluble gel was formed, but when a solution of 25 g SiGMA, 75 mg CGI 819 and 44 g isopropyl acetate was similarly irradiated for 1 hour at room temperature under nitrogen with Philips TL03 bulbs a clear, viscous polymer solution was formed. The solution was poured into 100 ml acetonitrile and the resulting top layer was decanted. 20 ml EtOAc was added to form a clear solution. 100 ml additional acetonitrile was added and the top layer was decanted. 35 ml EtOAc was added to the lower layer to form a clear solution and 100 ml acetonitrile was added to precipitate again. The top layer was decanted. 20 g D3O was added to the bottom layer, which was then stripped in a rotary evaporator to form 39.4 g of a clear, somewhat viscous solution of 49% prepolymer and 51 % D3O ("SiGMA Prepolymer Solution").

Example 10

A clear solution was formed from 4 g of the SiGMA Prepolymer Solution, 1.31 g DMA, 0.05 g TEGDMA and 10. μl DAROCUR 1173. Lenses were formed by curing in TOPAZ and polypropylene contact lens molds for 1 hour with a Philips 20W/09N UV fluorescent bulbs under nitrogen at room temperature. The molds were opened and the lenses were released and extracted in 70/30 IPA/water. (2 exchanges) and then placed in saline and autoclaved. The properties of these lenses are shown in Table 2.

Table 2

| | |
|---|---|
| Dk | 51 |
| Haze | 227 +/- 21% |
| Water content | 55.7 +/- 0.3% |
| Dynamic contact angle | 70 +/- 20° |

Example 11

SiGMA and CGI 819 where reacted following the procedure of Example 9, except that after the final precipitation the polymer was isolated as 10.5 g clear viscous liquid without addition of D3O. 1.16 g of this prepolymer was mixed overnight with 0.81 g NVP, 0.04 g TEGDMA, 0.03 g CGI 819 and 0.80 g EtOAc as diluent to form a clear solution. Lenses were formed by curing in ZEONOR and polypropylene contact lens molds for 1 hour with a Philips TL 20w/03T fluorescent bulbs under nitrogen at 60°C. The molds were opened and the lenses were released and extracted in IPA/water and then placed in saline. The hydrated lenses were moderately hazy.

Example 12

Following the procedure of Example 11, lenses were formed from 1.16 g SiGMA prepolymer, 0.79 g NVP, 0.03 g TEGDMA, 0.03 g CGI 819 and 0.82 g DPMA as diluent. The resulting hydrated lenses were slightly hazy.

Example 13

A mixture of 150 g of octamethylcyclotetrasiloxane, 22.6 g of (3,3,3-trifluoropropyl) methyl cyclotrisiloxane, 17.4 g of 1,3,5,7-tetramethylcyclotetrasiloxane, 0.05 g of hexamethyldisiloxane, 200 g of chloroform and 1.5 g of trifluoromethane sulfonic acid is stirred for 24 hours at 25° C., then washed repeatedly with purified water until a pH of the mixture becomes neutral. After water is separated, chloroform is distilled off under the reduced pressure. The residual liquid is dissolved in isopropanol, reprecipitated with methanol, followed by removal of volatile components under the vacuum from a separated liquid to give a transparent viscous liquid. The transparent viscous liquid is a polysiloxane having hydrosilane groups.

A mixture of 48 g of this polysiloxane, 11.6 g of allyl alcohol, 96 g of isopropyl alcohol, 0.04 g of potassium acetate, 10 mg of chloroplatinic acid and 10 mg of di-t-butylcresol is charged into a flask with a reflux condenser and heated with stirring for 3 hours at 50°C. The reaction mixture is filtered, then isopropanol is distilled off under reduced pressure, followed by washing with a mixture of methanol/water. Further removal of volatile components under the vacuum gives a transparent viscous liquid. The resulting liquid is a polysiloxane prepolymer having alcohol groups.

Example 14

A clear reactive mixture is formed from 55.5% (wt) polysiloxane prepolymer from Example 13, 39% DMA, 4% TEGDMA, 1.5 % CGI 819 with D3O in a ratio of 70/30 (weight ratio of prepolymer and reactive components to D3O diluent). This reactive mixture is degassed under 40 mm Hg for 20 minutes, then cured in TOPAZ front and polypropylene back contact lens molds under a Philips TL03 light for 1 hour at 5°C. The molds are opened and the lenses are released into a 50:50 (wt) solution of IPA and water. The lenses are extracted with IPA for 2 hours, then transferred (20 minutes in each) to 75:25, 50:50, 25:75 and 0:100 solutions of IPA and water.

## Claims

**1.** A polymer composition formed from a reactive mixture comprising

(a) at least one substantially non-reactive prepolymer having a weight average molecular weight from 50,000 Daltons to 1,000,000 Daltons as measured via gel permeation chromatography using refractive index detection and comprising less than 1 weight% reactive groups, said prepolymer comprising silicone containing groups and compatibilizing groups and
(b) a hydrophilic component comprising at least one monomer capable of hydrogen bonding with said prepolymer, provided that said reactive mixture is substantially free of reactive prepolymer, wherein

said prepolymer does not covalently bond to itself or other components in the reactive mixture during curing,
said reactive groups are groups capable of forming covalent bonds under the curing and processing conditions selected to make the polymer and any desired article therefrom and
said compatibilizing groups are selected from the group consisting of carboxyl groups, thiols, phenols, amides of primary amines, ammonia, ureas of primary amines, urethanes of primary amines, hydroxyl groups and combinations thereof.

**2.** The polymer of claim 1 wherein said prepolymer comprises at least about 50 weight% groups comprising fluorine, silicone or a mixture thereof.

**3.** The polymer of claim 1 wherein said hydrophilic component comprises at least one amide containing monomer.

**4.** The polymer of claim 2 wherein said hydrophilic component comprises at least 50 weight% amide containing monomer.

**5.** The polymer of any preceding claim wherein said hydrophilic component comprise at least one hydrogen bond participant.

**6.** The polymer of any preceding claim wherein said compatibilizing group is a hydrogen bond donating group.

**7.** The polymer of claim 1 wherein said polymer has an oxygen permeability of at least 30 barrer.

**8.** The polymer of claim 1 wherein said hydrophilic component comprises at least one amide monomer, urethane monomer or mixtures thereof.

**9.** The polymer of claim 1 wherein said hydrophilic component is selected from the group consisting of N,N-dimethyl acrylamide, 2-hydroxyethyl methacrylamide, N-vinylpyrrolidone, N-vinyl-N-methylacetamide, N-vinylacetamide, N-vinyl-N-methylpropionamide, N-vinyl-N-methyl-2-methylpropionamide, N-vinyl-2-methylpropionamide, N-vinyl-N,N'-dimethylurea, and mixtures thereof.

**10.** The polymer of claim 1 wherein said hydrophilic component is selected from the group consisting of N,N-dimethyl

acrylamide, N-vinylpyrrolidone, N-vinyl-N-methylacetamide and mixtures thereof.

**11.** A contact lens formed from the polymer of any one of claims 1 to 10.

**12.** A biomedical device formed from the contact lens of claim 11.

**Patentansprüche**

**1.** Polymerzusammensetzung, gebildet aus einem reaktionsfähigen Gemisch umfassend

(a) wenigstens ein im Wesentlichen nichtreaktives Präpolymer mit einem gewichtsgemittelten Molekulargewicht von 50.000 Dalton bis 1.000.000 Dalton, wie gemessen durch Gelpermeationschromatographie unter Verwendung von Brechungsindexnachweis, und umfassend weniger als 1 Gew.-% an reaktionsfähigen Gruppen, wobei das Präpolymer Silicon-enthaltende Gruppen und Kompatibilisierungsgruppen umfasst, und
(b) eine hydrophile Komponente, umfassend wenigstens ein Monomer, das zu Wasserstoffbrücken mit dem Präpolymer fähig ist, vorausgesetzt, dass das reaktionsfähige Gemisch im Wesentlichen frei von reaktionsfähigem Präpolymer ist, wobei

das Präpolymer während des Härtens nicht an sich selbst oder andere Komponenten in dem reaktionsfähigen Gemisch kovalent bindet,
die reaktionsfähigen Gruppen fähig sind, unter den ausgewählten Härtungs- und Verarbeitungsbedingungen kovalente Bindungen zu bilden, um das Polymer und einen gewünschten Gegenstand daraus herzustellen, und die Kompatibilisierungsgruppen ausgewählt sind aus der Gruppe bestehend aus Carboxygruppen, Thiolen, Phenolen, Amiden von primären Aminen, Ammoniak, Harnstoffen von primären Aminen, Urethanen von primären Aminen, Hydroxygruppen und Kombinationen davon.

**2.** Polymer gemäß Anspruch 1, wobei das Präpolymer wenigstens 50 Gew.-% an Gruppen umfasst, die Fluor, Silicon oder ein Gemisch davon umfassen.

**3.** Polymer gemäß Anspruch 1, wobei die hydrophile Komponente wenigstens ein Amid-enthaltendes Monomer umfasst.

**4.** Polymer gemäß Anspruch 2, wobei die hydrophile Komponente wenigstens 50 Gew.-% an Amidenthaltendem Monomer umfasst.

**5.** Polymer gemäß einem der vorstehenden Ansprüche, wobei die hydrophile Komponente wenigstens einen Wasserstoffbrückenbildner umfasst.

**6.** Polymer gemäß einem der vorstehenden Ansprüche, wobei die Kompatibilisierungsgruppe eine Wasserstoffbrücken-Donatorgruppe ist.

**7.** Polymer gemäß Anspruch 1, wobei das Polymer eine Sauerstoffpermeabilität von wenigstens 30 Barrer aufweist.

**8.** Polymer gemäß Anspruch 1, wobei die hydrophile Komponente wenigstens ein Amidmonomer, Urethanmonomer oder Gemische davon umfasst.

**9.** Polymer gemäß Anspruch 1, wobei die hydrophile Komponente ausgewählt ist aus der Gruppe bestehend aus *N,N*-Dimethylacrylamid, 2-Hydroxyethylmethacrylamid, N-Vinylpyrrolidon, N-Vinyl-*N*-methylacetamid, N-Vinylacetamid, N-Vinyl-N-methylpropionamid, N-Vinyl-N-methyl-2-methylpropionamid, N-Vinyl-2-methylpropionamid, N-Vinyl-*N,N*'-dimethylharnstoff und Gemischen davon.

**10.** Polymer gemäß Anspruch 1, wobei die hydrophile Komponente ausgewählt ist aus der Gruppe bestehend aus N,N-Dimethylacrylamid, N-Vinylpyrrolidon, N-Vinyl-*N*-methylacetamid und Gemischen davon.

**11.** Kontaktlinse, gebildet aus dem Polymer gemäß einem der Ansprüche 1 bis 10.

**12.** Biomedizinische Vorrichtung, gebildet aus der Kontaktlinse gemäß Anspruch 11.

## Revendications

**1.** Composition polymère formée d'un mélange réactif comprenant

(a) au moins un prépolymère substantiellement non réactif de masse moléculaire moyenne en masse comprise entre 50 000 daltons et 1 000 000 daltons comme mesuré par chromatographie à perméation de gel en utilisant la détection par indice de réfraction et comprenant moins de 1 % en masse de groupements réactifs, ledit prépolymère comprenant des groupements contenant de la silicone et des groupements compatibilisants et
(b) un composant hydrophile comprenant au moins un monomère capable d'engager des liaisons hydrogène avec ledit prépolymère, à la condition que ledit mélange réactif soit essentiellement exempt de prépolymère réactif, où

ledit prépolymère ne se lie pas de façon covalente à lui-même ou d'autres composants dans le mélange réactif pendant le durcissement,
lesdits groupements réactifs sont des groupements capables de former des liaisons covalentes dans les conditions de durcissement et de transformation choisies pour fabriquer le polymère et n'importe quel article souhaité à partir de celui-ci et
lesdits groupements compatibilisants sont choisis dans le groupe constitué par les groupements carboxyle, les thiols, les phénols, les amides d'amines primaires, l'ammoniac, les urées d'amines primaires, les uréthanes d'amines primaires, les groupements hydroxyle et leurs combinaisons.

**2.** Polymère selon la revendication 1, où ledit prépolymère comprend au moins environ 50 % en masse de groupements comprenant du fluor, de la silicone ou l'un de leurs mélanges.

**3.** Polymère selon la revendication 1, où ledit composant hydrophile comprend au moins un monomère contenant un amide.

**4.** Polymère selon la revendication 2, où ledit composant hydrophile comprend au moins 50 % en masse d'un monomère contenant un amide.

**5.** Polymère selon l'une quelconque des revendications précédentes, où ledit composant hydrophile comprend au moins un participant à une liaison hydrogène.

**6.** Polymère selon l'une quelconque des revendications précédentes, où ledit groupement compatibilisant est un groupement donneur de liaison hydrogène.

**7.** Polymère selon la revendication 1, où ledit polymère présente une perméabilité à l'oxygène d'au moins 30 barrer.

**8.** Polymère selon la revendication 1, où ledit composant hydrophile comprend au moins un monomère contenant un amide, un monomère contenant un uréthane ou leurs mélanges.

**9.** Polymère selon la revendication 1, où ledit composant hydrophile est choisi dans le groupe constitué par les suivants : N,N-diméthylacrylamide, 2-hydroxyéthylméthacrylamide, N-vinylpyrrolidone, N-vinyl-N-méthylacétamide, N-vinylacétamide, N-vinyl-N-méthylpropionamide, N-vinyl-N-méthyl-2-méthylpropionamide, N-vinyl-2-méthylpropionamide, N-vinyl-N,N'-diméthylurée, et leurs mélanges.

**10.** Polymère selon la revendication 1, où ledit composant hydrophile est choisi dans le groupe constitué par les suivants : N,N-diméthylacrylamide, N-vinylpyrrolidone, N-vinyl-N-méthylacétamide, et leurs mélanges.

**11.** Lentille de contact formée à partir du polymère selon l'une quelconque des revendications 1 à 10.

**12.** Dispositif biomédical formé à partir de la lentille de contact selon la revendication 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03022322 A
- US 3808178 A
- US 4120570 A
- US 4136250 A
- US 4153641 A
- US 4740533 A
- US 5034461 A
- US 5070215 A
- US 5998498 A
- EP 080539 A
- US 4910277 A
- US 6822016 B
- US 3408429 A
- US 3660545 A
- US 4113224 A
- US 4197266 A
- US 4495313 A
- US 4680336 A
- US 4889664 A
- US 5039459 A


### Non-patent literature cited in the description


- Photoinitiators for Free Radical Cationic & Anionic Photopolymerization. John Wiley and Sons, 1998, vol. III
- Handbook of Polymer Liquid Interaction Parameters and Solubility Parameter. CRC Press, Inc, 1990
- **A. F. M. BARTON.** Handbook of Solubility Parameters and Other Cohesion Parameters. CRC Press, 1985
- Hansen Solubility Parameters. **CHARLES M. HANSEN.** A User's Handbook. CRC Press, 2000, 43-53